# EUROPEAN PATENT APPLICATION

(11) **EP 4 286 854 A2**
(43) Date of publication of application: **06.12.2023**
(21) Application number: 23461600.1
(22) Date of filing: 31.05.2023
(51) Int. Cl.: G01N 33/574

(54) **SET OF PROTEIN BIOMARKERS AND METHOD FOR DIAGNOSING COLORECTAL CANCER IN VITRO**

(30) Priority: 31.05.2022 PL 44131922
(71) Applicant: Uniwersytet Medyczny Im. Piastów Slaskich We Wroclawiu, 50-367 Wroclaw (PL)
(72) Inventor: Dziegiel, Piotr, 51-650 Wroclaw (PL); Zadka, Lukasz, 56-400 Olesnica (PL); Rusak, Agnieszka, 50-512 Wroclaw (PL)
(74) Representative: Witek, Rafal

(57) **Abstract**

A subject of the invention is a set of protein biomarkers for *in vitro* diagnosis of early stage colorectal cancer or colorectal cancer comprising TIMP-1, TIMP-2, IL8, GRO-α, HGF and Ang-1. A further subject of the invention is a method for *in vitro* diagnosis of early stage colorectal cancer or colorectal cancer comprising assessment of expression levels in extracellular vesicles of at least two biomarkers from the group comprising TIMP-1, TIMP-2, IL8, GRO-α, HGF and Ang-1.

## Description

The subject of the invention is a set of six protein biomarkers such as angiopoietin 1 (ANG-1), inhibitor of metalloproteinase 1 (TIMP-1), inhibitor of metalloproteinase 2 (TIMP-2), interleukin 8 (IL-8), growth-regulated oncogene alpha (GRO-α), hepatocyte growth factor (HGF), as well as a method for *in vitro* diagnosis of colorectal cancer. The invention relates to the field of oncology diagnostics.

From European patent application EP2841947 A1, a method of diagnosing colorectal cancer is known, which comprises testing a biological sample for one or more biomarkers selected from a defined group, whereby the presence or increased expression of one or more of the biomarkers indicates that a person is at risk of colorectal cancer or has colorectal cancer. From international patent application WO2020225426 A1, a method of diagnosing colorectal cancer is known, which comprises determining the level of at least one, and preferably 3-4, and most preferably 5 protein biomarkers selected from the group of protein biomarkers AREG, CEA, IGFBP2, keratin, KRT19, MASP1, OPN, PON3 and TR.

A biomarker for the detection of early stage colorectal cancer is known from European patent application EP3647788 A1, which biomarker is selected from the group of biomarkers i.e. annexin A11, annexin A3, annexin A4, tanascin-N, transferrin receptor protein 1, glucose transporter 1, complement component C9, CD88 antigen, 78 kDa glucose-regulated protein, α-1-acid glycoprotein, matrix metalloproteinase 9, angiopoietin-1, CD67 antigen, mucin-5B, GRB2 adaptor protein, annexin A5, olfactomedin-4, neutral amino acid transporter B(0), tripeptidylpeptidase 1, heat shock-related 70 kDa protein 2, proteasome subunit α type 5, neutrophil gelatinase-associated lipocalin.

From the literature publication entitled "Diagnostic values of MMP-7, MMP-9, MMP-11, TIMP-1, TIMP-2, CEA, and CA 19-9 in patients with colorectal cancer" by Xiwen Huang, Yongquan Lan, En Li, Jiaquan Li, Qiaoting Deng, Xunwei Deng, studies on the evaluation of MMP-7, MMP-9, MMP-11, TIMP-1, TIMP-2, CEA, and CA19-9 biomarkers in the diagnosis of colorectal cancer are known.

In the current state of the art, there are a number of as yet unsolved technical problems, such as too late diagnosis of colorectal cancer due to the relatively late symptomatisation of the cancer, which prevents rapid treatment initiation, or invasive diagnosis of patients with suspected colorectal cancer by colonoscopy, which is the gold standard diagnostic procedure, or rectoscopy, where there is a risk of mechanical damage to the bowel, perforation, bleeding and other complications, or even death of the patient through inappropriate endoscopic procedure. For the early detection of colorectal cancer, pathologically altered tissue is directly sampled, and the tissue sampling itself is strictly dependent on the skill and experience of the medical professionals performing the biopsy, so there is a possibility of intra-intestinal infection in the course of the aforementioned invasive diagnostic procedures. In addition, in the current state of the art, molecular diagnostic methods are characterised by low diagnostic efficiency for the detection of colorectal cancer, where negative laboratory results are often obtained, indicating no pathological features, which definitively hinders the initiation of an anti-cancer therapy tailored to the patient. To date, there is also a lack of protein biomarkers of recognised efficacy applicable to the routine detection of colorectal cancer. According to the current state of the art, it is also impossible to identify single biomarkers with high sensitivity and specificity for detecting cancerous lesions in the gastrointestinal tract. An ideal solution to the problem at hand would be to detect several different biomarkers or extracellular structures containing relevant biomarkers and released by the tumour cells, the molecular parameters of which would reflect the unique biochemical processes and metabolism occurring in cancer, different from that of normal tissue. Currently, the most common diagnostic standard is to sample pathologically altered tissue at a specific site in the colon for histopathological evaluation of the tissue fragments secured. The problem with commonly used diagnostic tools is not only their invasiveness, but also the need for adequate preparation of the patient before the planned procedure and the inconvenience and lack of comfort during endoscopic procedures. However, one of the most important limitations of endoscopic examinations is the lack of specific symptoms of gastrointestinal tumours, including colorectal cancer, with the result that patients present for diagnostic purposes most often at a late stage of the disease with an already advanced form of the tumour, when complete cure is usually very difficult or impossible; thus, diagnosis of the tumour at this stage by histopathological examination rarely translates into prognostic value and better survival times for patients because of the advanced tumour process, local malignancy of the tumour (infiltration of neighbouring tissues), inability to perform a complete resection (removal) of the tumour lesion and the presence of distant metastases to other organs.

Unexpectedly, all the technical problems indicated above have been solved by the present invention.

A subject of the invention is a set of protein biomarkers for *in vitro* diagnosis of early stage colorectal cancer or colorectal cancer, characterised in that it comprises TIMP-1, TIMP-2, IL8, GRO-α, HGF and Ang-1.

Preferably, the set of protein biomarkers is characterised in that the expression value of any biomarker of the group comprising TIMP-1, TIMP-2, IL8, GRO-α, HGF, Ang-1 is determined in extracellular vesicles.

Another subject of the invention is a method for *in vitro* diagnosis of early stage colorectal cancer or colorectal cancer, characterised in that it comprises the following steps:
a) a biological sample in the form of tissue material is obtained from the patient,
b) extracellular vesicles are isolated from the biological sample obtained from the patient,
c) in the isolated extracellular vesicles, the expression levels of at least two protein biomarkers selected from a set including TIMP-1, TIMP-2, IL8, GRO-α, HGF and Ang-1 are determined.

Preferably, the method is characterised in that the tissue material is solid tissue or body fluid. Preferably, the method is characterised in that the body fluid may be blood, plasma, serum, saliva or urine.

Preferably, the method is characterised in that the extracellular vesicles are exomers, exosomes, ectosomes, microvesicles, apoptotic bodies or oncosomes.

Preferably, the method is characterised in that the isolation of extracellular vesicles is carried out by centrifugation, ultracentrifugation, chromatography or by using kits for precipitation of extracellular vesicles.

Preferably, the method is characterised in that ELISA immunoassay method, immunofluorescence assay method, Western blot (immunoblot) method, flow cytometry method, flow nanocytometry method, protein microarray method or detection using biosensors is used to evaluate the biomarker expression level.

Preferably, the method is characterised in that the expression levels of the TIMP-1, TIMP-2, IL8, GRO-α and HGF biomarkers correspond to their elevated concentrations in a patient with early stage colorectal cancer or colorectal cancer than in a healthy person.

Preferably, the method is characterised in that the expression level of the Ang-1 biomarker corresponds to its reduced concentration in a patient with early stage colorectal cancer or colorectal cancer than in a healthy person.

The aim of the present invention is early diagnosis of cancer in individuals with suspected colorectal cancer, enabling correct diagnosis of a patient with suspected cancer and initiation of appropriate treatment. Another aim is to introduce a modern form of performing a minimally invasive diagnosis in individuals with suspected colorectal cancer instead of an invasive, inconvenient colonoscopy etc. Another aim of the invention is to completely eliminate the risk of mechanical damage to the bowel, perforation, bleeding and other complications or even death by reducing the use of colonoscopy or rectoscopy, where blood, plasma or urine is collected for diagnostic purposes according to the invention to obtain extracellular vesicles (including those derived from cancer cells). Another aim is to increase the survival time of patients diagnosed with colorectal cancer and to improve their quality of life through earlier detection of cancerous lesions at an earlier clinical stage. The aim of the invention is also to provide a high diagnostic efficiency for the detection of colorectal cancer in patients, by being able to isolate extracellular tumour vesicles regardless of where the biological sample is taken. The aim is also to be able to assess a wide variety of protein biomarkers, carried even in single extracellular vesicles, which are released unchanged directly from the tumour cells, contain a unique composition and level of given biomarkers, enabling the detection and demonstration of differences between a cancer and a healthy person.

The invention provides a set of protein biomarkers and an *in vitro* diagnostic method for the early detection of colorectal cancer, which includes the use of the biomarkers and a method for the preparation of biological samples obtained from a patient, allowing demonstration of significant differences in their expression at the protein level, which enables a more efficient detection of selected molecules and a more accurate demonstration of changes in their concentration levels depending on the diagnosis. Extracellular vesicles (EVs) are structures released by cells into the external environment, whose lumen, surrounded by a phospholipid bilayer, provides the transport of numerous biomolecules and their assimilation into target cells [1]. The extracellular environment here is specifically body fluids such as blood and the serum and plasma derived from it, cerebrospinal fluid, saliva, urine and other secretions containing products of the biological activity of specific cells, since the ability to release extracellular vesicles is a feature of all mammalian cells, making them a common component of many different tissues [1]. Biomolecules transported in EVs as their cargo consist of numerous nucleic acid, lipid and protein molecules, the composition of which changes based on tissue metabolism, and the quantitative and qualitative changes in EV cargo that occur are a unique pattern reflecting the specificity and molecular activity of vesicle-releasing cells [1,2]. The phospholipid envelope protects the transported cargo from digestion by proteases present in the systemic circulation, thus ensuring the safe transit of intact biomolecules, even over considerable distances from the original site of vesicle release, which is currently being exploited in the detection of EVs as a reservoir of novel biomarkers of selected diseases, particularly cancer [3]. EVs released by tumour cells have the ability to transport oncogenes, are associated with cancer progression and have been shown to be associated with pre-metastatic niche formation, which increases the risk of distant metastasis, in selected internal organs; they may also contain a unique set of biomarkers allowing to demonstrate their specific tumour cell origin [4, 5, 6]. The update of the current definition of a tumour biomarker emphasises the need to include in the diagnosis a whole set of transcripts or proteins whose clinical value exceeds the evaluation of single molecules [7]. The unique content of EVs and their biological activity address the current needs of practical medicine and highlight the potential of extracellular vesicles as novel cancer biomarkers. Harnessing the diagnostic potential of EVs and, in particular, exosomes appears to be of increasing importance in colorectal cancer (CRC).

The practical application of EVs in the early diagnosis of solid tumours seems to be most valid for the polypeptides carried in their cargoes, rather than nucleic acids. According to the observations of Zheng *et al*. the use of recently popular miRNA molecules in diagnostic panels is of limited practical use due to the relatively low percentage of miRNAs versus complete noncoding RNA in extracellular vesicles, while proteins in the category of novel EVs biomarkers related to cancer detection may be a better alternative [8]. Furthermore, the authors noted that EVs secreted by tumour cells are present in the systemic circulation of a patient diagnosed with CRC, and their isolation from plasma and the biochemical analyses performed show a different proteome than in the control group tested, allowing the use of EVs in the early, non-invasive diagnosis of this cancer [8]. The presence of tumour-associated EVs in the body fluids is a widely documented and well-studied phenomenon in the literature to date. The increased content of tumour-associated EVs in the systemic circulation and the associated changes in the abundance of tumour-specific extracellular vesicles may be the result of their increased release by tumour cells, which are the predominant tissue component of cancer. In our studies to date, we have shown that exosomal tetraspanins are overexpressed in CRC tumour cells, and that the nanoparticles corresponding to EVs extracted from cancer tissue fragments are at significantly higher concentrations than in tumour-adjacent normal control tissue [9]. Changes in the optical parameters of EVs also occur at an early stage of colorectal cancer, both in the refractive index and in the mass density of individual vesicles, which may imply that the changes occurring in EVs loads are related to the process of carcinogenesis towards CRC and express the altered biochemical composition (biomarkers) of tumour EVs released into the blood from the altered tumour tissue [10]. To date, no protein biomarkers of EVs with recognised potential for practical use in CRC diagnosis have been proposed, nevertheless, proteomic analyses have detected differences in the proteome between the control group and the clinical stage of CRC in the blood serum of the patients studied, highlighting the possibility of minimally invasive detection of this cancer using vesicle-mediated proteins [11]. Numerous mediators of the acute and chronic immune response through chemotaxis modulate the properties of blood vessels by regulating, among other, their permeability in order to restore the original state of homeostasis [12].

In certain circumstances, under both physiological and pathological conditions, the formation of new thin-walled blood vessels on the basis of the already existing vascular bed occurs, which is a complex and intricate process that proceeds in multiple stages and is variously modifiable in relation to the tissue-specific location, the causes and the current stage of the process [13]. During postnatal life, the process described is most often pathological in origin and is particularly involved in the genesis of tumours, where the balance between proangiogenic factors and those that act to inhibit the formation of a new vascular bed is disturbed and determines tumour growth [14]. The importance of angiogenesis in cancer pathology was first described by Judah Folkman, who recognised the importance of this process in promoting progression and metastasis [15]. Folkman noted the significant acceleration of neovascularisation during active carcinogenesis, where its intensity largely exceeded the inflammation-induced rate of vascularisation occurring during the compensatory regenerative processes of wound healing - furthermore, he revealed the centrality of neovascularisation to solid carcinomas early in their development, as a necessary phenomenon for exceeding the critical size of the tumour focus beyond the 2-3 mm range [16]. This provides an opportunity to use pro-angiogenic factors for the early detection of tumours. Angiogenesis and the immune response are interdependent processes, and their vital importance in regulating the tumour process is a fact that is difficult to dispute. For a long time, pro-angiogenic activity has been associated with overactivity of pro-inflammatory factors - during the process of wound healing, local inflammation or eventually an inflammation-driven tumour focus, immune-activating factors show significant effects on vascular wall properties and on endothelial cell function and characteristics. Cytokines and chemokines are a broad group of different proteins with multipotent properties, regulating numerous cell signalling pathways, with leucocytes being the main source of production, but their secretion also occurs in other somatic cells [17]. The ability to release cytokines is also demonstrated by germinal cells, with interleukins (ILs) released by these cells belonging to this category of proteins that present properties of shaping anticancer immunity [18,19]. Elevated levels of pro-inflammatory cytokines have been reported in numerous cancers, which show a correlation with increased cancer risk, disease progression, clinical tumour stage, or response to treatment [20,21]. The obvious link between tumour-induced angiogenesis and inflammation, which most cytokines are capable of modifying, makes it reasonable to hypothesise whether and to what extent pro-angiogenic and pro-inflammatory cytokines transported in EVs can be used for the early detection of CRC. The growth-regulated oncogene (GRO) cytokine belongs to the family of chemokines with a CXC motif at the NH₂-containing end of the chain such as the GRO-α, GRO-β and GRO-γ proteins, the expression of which may already be altered at an early stage of colorectal cancer development corresponding to the polyp stage [22]. The level of GRO-α (CXCL1) cytokine in tissue homogenates of colorectal adenocarcinomas was significantly higher in cancer than in normal controls [23], reaching mean concentrations values of 395.7±133.6 pg/g protein in colorectal mucosa and 2430±789.4 pg/g protein in the tumours studied (P=0.03). GRO-α as well as interleukin IL8 (CXCL8) are among the growth factors with potential pathogenetic significance in CRC [23]. Baier *et al*. showed that also IL8 levels in prepared CRC homogenates were significantly higher in cancer (5126±1521 pg/g) than in normal colorectal mucosa (74±20.49 pg/g) with P value of 0.0087 [23]. The importance of tissue inhibitors of metalloproteinases (TIMPs) in CRC remains poorly understood to date. TIMPs are among the inhibitors of metalloproteinase activity responsible for extracellular matrix remodelling, and expression of TIMP-1 has been reported more frequently in mononuclear inflammatory cells (MICs) in patients with Crohn Disease (CD) with malignant transformation to CRC [24]. These findings may suggest the importance of TIMP-1 in the early stage of carcinogenesis of the CRC subtype, in which pathogenesis is associated with chronic inflammation. Inflammatory bowel diseases (IBD) such as CD and ulcerative colitis (UC) are among the known pre-cancerous conditions with a relatively high risk of transformation to CRC.

In IBD, colorectal cancer as a complication of the disease is also the most common cause of death reaching up to 15% of all patients, and chronic colon inflammation (pancolitis) is one of the main risk factors for carcinogenesis towards CRC [25]. According to recent reports, TIMP proteins appear to have prognostic significance in CRC patients, showing an association with biological tumour malignancy. Using enzyme-linked immunosorbent assay (ELISA), significantly higher serum levels of TIMP-1 and TIMP-2 were confirmed in CRC patients as compared to the control group (P<0.01). TIMP-1 and TIMP-2 levels were increased in patients with higher CRC stage and in patients who had lymph node involvement (P<0.001). TIMP-1 levels were higher in patients with distant metastases (P<0.001), while higher TIMP-2 concentrations were reported in older patients (P=0.004) [26]. Another biomarker of prognostic importance in CRC is Hepatocyte Growth Factor (HGF, DFNB39, HPTA) known to regulate the motility and proliferative activity of epithelial cells [27]. In colorectal cancer, overexpression of HGF has been associated with reduced overall survival (OS) and disease-free survival (DFS) [27]. Angiopoietin-1 (ANG-1) is one of the vascular growth factors that regulate neovascularisation and angiogenesis by interacting as a ligand with the Tie-2 receptor [28]. The Ang-1/Tie-2 signalling pathway maintains the stability of mature blood vessels and prevents endothelial cell activation by pro-inflammatory and pro-angiogenic cytokines circulating in the extracellular milieu [29]. Significantly higher plasma levels of Tie-2 receptor (median 22 ng/mL, minimum value: 12, maximum value: 46) were found in CRC patients by ELISA and no differences for Ang-1 concentrations between tumour and control groups [30]. Angiostatin, on the other hand, is one of the endogenous inhibitors of angiogenesis, whose biological activity may have potential applications in anticancer therapy [31]. The importance of angiostatin in the pathogenesis of colorectal cancer is as yet poorly understood. Patients with CRC who underwent minimally invasive colorectal resection (MICR) had overexpression of proangiogenic factors in the postoperative period [32]. Angiotensin levels determined in the plasma of CRC patients using ELISA were not significantly different between the preoperative period and after tumour resection [32], thus angiostatin did not affect the proangiogenic activity resulting from MICR. Also, studies conducted in animal models of CRC did not demonstrate a significant antitumour effect of angiotensin [33].

A set of six protein markers and a method of assaying them designed for the early detection of colorectal cancer provides analysis of altered protein expression of Ang-1, TIMP-1, TIMP-2, IL-8, GRO-α and HGF and differentiates normal from cancer tissue. In cancer, there is an increased expression of TIMP-1, TIMP-2, IL8, GRO-α and HGF, while Ang-1 levels become reduced. The method for the determination of the aforementioned biomarkers relates to their detection on pre-isolated extracellular vesicles (EVs), in particular of the exosome subtype, extracted from the biological samples under investigation, whereby the solution according to the invention is not limited to exosomes only, and extracellular vesicles may also represent exomers, ectosomes, microvesicles, apoptotic bodies or oncosomes. Various methods for the isolation of vesicles, also exosomes, have been described, including differential centrifugation, ultracentrifugation, chromatography or vesicle isolation using commercially available reagents that facilitate EV precipitation and formation of a pellet layer. The preferred biological sample is venous blood collected from the patient and plasma and serum fractions extracted from it, but also urine and saliva. The biological sample tested can also be solid tissue, e.g. a tissue biopsy taken endoscopically or surgically during resection of the diseased colon wall. The invention involves assessment of the levels of at least two of the above-mentioned polypeptides by immunoenzymatic ELISA, by Western blot, by flow cytometry and flow nanocytometry (nFCM) methods, as well as by using chips in protein microarray technology and other biosensors. An antigen-antibody reaction or a modification thereof to assess the levels of the analyzed biomarkers is the essence of detection. The evaluation of biomarkers in extracellular vesicles allows for detection of significant differences in their concentrations due to the diverse expression of these proteins between tumour and normal conditions. The varying content of biomarkers in EVs shows diagnostic value for patients diagnosed with colorectal cancer, and their assessment will facilitate the early diagnosis of this widespread cancer showing a lack of specific symptoms to direct the physician to make an appropriate and early diagnosis. Early diagnosis of CRC using the aforementioned biomarkers will also contribute to improving the health of the general population by increasing the detection rate of CRC and providing for the initiation of appropriate therapy at an early stage of the cancer.

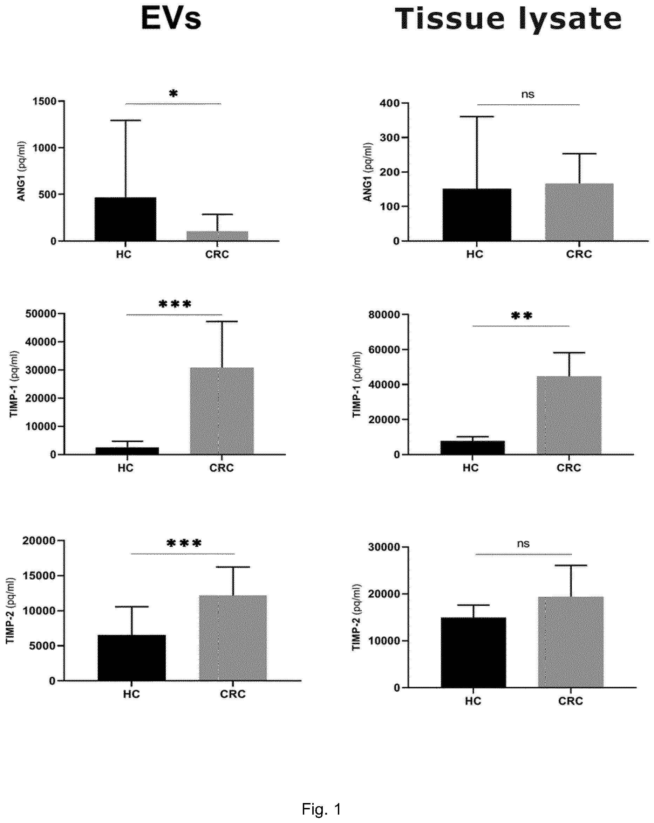

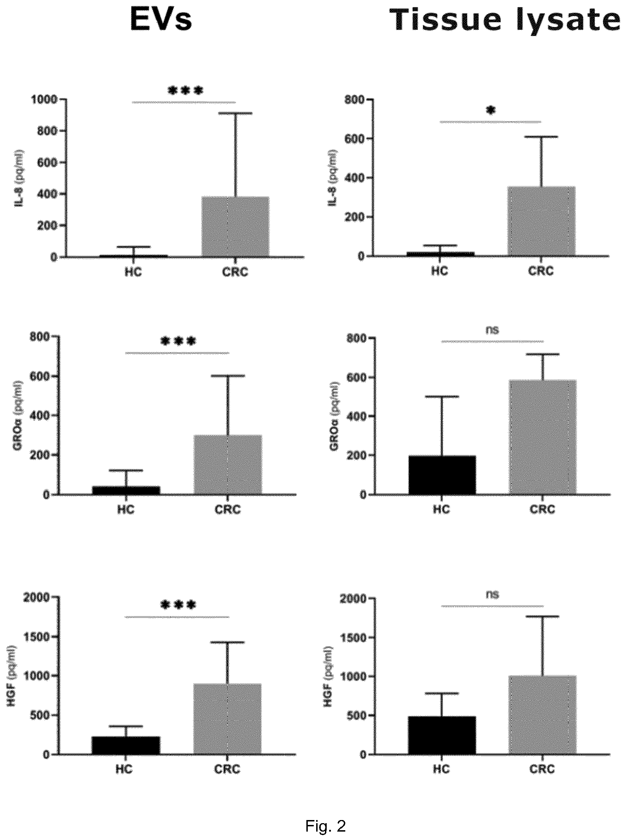

An embodiment of the subject of the invention is illustrated in the drawing, in which Fig. 1 shows the change in concentration of protein biomarkers ANG1, TIMP-1, TIMP-2 in extracellular vesicles and in tissue lysates from healthy subjects and patients with colorectal cancer, Fig. 2 shows the change in concentration of protein biomarkers IL-8, GRO-α, HGF in extracellular vesicles and tissue lysates from healthy patients and patients with colorectal cancer, with HC denoting control, CRC denoting colorectal cancer, EVs denoting extracellular vesicles, 'ns' denoting non-significant sample, * denoting P≤0.05, ** denoting P<0.01, *** denoting P<0.001.

Extracellular vesicles (EVs) were isolated from the same tissue fragments as the prepared tissue lysates, wherein the tissue lysate here is a complete tissue containing the actual tumour cells, the inflammatory cells infiltrating the tumour, the cellular elements of the vasculature, the connective tissue elements constituting the tumour lining and the extracellular vesicles themselves being the product of the previously mentioned cellular structures. The term EVs, on the other hand, refers only to the isolated extracellular vesicles contained in the tissue and does not include other components thereof (cells and other elements). The embodiment highlights the differences in the levels of biomarkers tested between the EVs alone extracted from tissue fragments and the whole complex tissue analysed, and demonstrates the presence of more pronounced differences in the biomarker expression assessed in the isolated EVs alone.

The invention includes a set of biomarkers detected in extracellular vesicles. Tumour-derived extracellular vesicles are expected to exhibit significant changes in the expression levels of the evaluated biomarkers compared to extracellular vesicles released by normal cells and in comparison to unpurified biological samples containing other components (cells, connective tissue elements, and others) in addition to extracellular vesicles. The release of tumour-associated extracellular vesicles into the extracellular space allows for the detection of these vesicles also in other tissue compartments, due to the increased distribution of extracellular vesicles released by tumour cells in the patient's systemic circulation. In extracellular vesicles, it is possible to determine numerous biomarkers simultaneously, as these structures transport and contain many different biomolecules reflecting the molecular characteristics and biochemical composition of the cells releasing them.

### Example 1 - Diagnostic materials and methods

EVs previously isolated from plasma by differential centrifugation and exosome precipitation using commercially available ExoQuick and Invitrogen reagents, as well as EVs isolated from solid tissue by classical, differential ultracentrifugation using sucrose cushion, including differential centrifugation using the ExoQuick method, showed significant heterogeneity depending on the clinical diagnosis (disease diagnosis) revealing morphometric differences including different vesicle diameters, as well as altered optical parameters of EVs allowing differentiation of their origin between colorectal cancer, histological malignancy grade, proliferative activity and a pre-cancerous condition - ulcerative colitis. Moreover, the optical parameters of EVs changed between the histological grade of the tumour as well as differing in elemental composition, implying a change in the charge of vesicles isolated from both body fluids and tissue isolates dependent on the diagnosis and grade of the tumour [10]. The change in vesicle charge may have resulted from both qualitative and quantitative changes in the levels of transported polypeptides (proteins), which are potentially valuable biomarkers of colorectal cancer and whose altered levels were responsible for the observed differences. Tumour cells are characterised by increased biogenesis of EVs in comparison to the normal colon wall, hence solid tissue (biopsy specimens) is the richest reservoir of tumour-associated EVs [9]. EV isolation was performed using tissue sections prepared from solid frozen tissue of paired colorectal cancer specimens and a corresponding normal control margin of the colon wall with healthy mucosa taken from a patient during surgical removal of the colorectal tumour. Tissue lysates were prepared from the respective tissue sections used to isolate EVs. The EVs extracted from the tissue are, under *in vivo* conditions, also released into the extracellular environment and constitute the common content of all vesicles present in the systemic circulation. Control material was collected during tumour resection from normal tissue margins, and additional microscopic verification of the collected mucosa was performed on prepared histological slides of 4 micrometres/slide thickness using standard haematoxylin and eosin slide staining - the absence of tumour cells and normal microanatomy of the mucosa and colon wall were the essential criteria for the inclusion of relevant samples in further study procedures. Tissue fragments (n = 32) were collected from 16 patients with histological diagnosis of CRC. None of the included patients had chemo- or radiotherapy initiated prior to the surgery. The detailed clinicopathological characteristics of the patients included in the study are shown in the table (Table 1).

| **Table 1.** Clinicopathological characteristics of the patients | | |
|---|---|---|
| **Parameter** | **Study group** | **Control group** |
| | CRC (n = 16) | HC (n = 16) |
| Age in years (range) | 69.69±8.882 | |
| Gender (F/M) | 5/11 | |
| Diagnosis *(Adenocarcinoma*/*Carcinoma)* | 13/3 | n/a |
| Grade (G₁/G₂/G₃) | 3/11/2 | n/a |
| TNM class (1/2/3/4) | 4/3/7/2 | n/a |
| Abbreviations: | | |
| CRC - colorectal cancer | | |
| HC - normal control/ normal colonic mucosa | | |
| F - female | | |
| M - male | | |
| n/d - not applicable | | |
| Grade (G1/G2/G3) - degrees of histological malignancy of the tumour indicating the degree of differentiation, high (G1), intermediate (G2) and poorly differentiated (G3) | | |
| TNM class - expresses the clinical stage of the tumour, where T represents the tumour, N represents the lymph node and M represents the metastasis | | |

For the preparation of EVs isolates, 65 mg of each frozen tissue measured with an electronic balance was collected into sterile Eppendorf-type tubes. Isolation of EVs was performed according to a previously published protocol [9] using immersion of tissue fragments in culture medium with additional digestion of the tissue with collagenase at 37°C while gently shaking the contents of the tubes. An appropriate volume of protease and phosphatase inhibitor cocktail was added to the solution according to the published protocol [9]. Sample purification and isolation of EVs was performed by differential centrifugation in successive cycles of 300 × G (5 minutes), 2000 × G (10 minutes) and 10000 × G (30 minutes) at 4°C. Each time, the supernatant obtained was preserved in new Eppendorf tubes and the pellet obtained was disposed of. The prepared EV isolates were stored at T = -80°C until further assay procedures were performed. In this study, 32 EV isolates were prepared representing paired surgical margin-cancer tissue pairs collected from 16 patients diagnosed with CRC. In addition to the EV isolates, 12 tissue lysate samples from CRC taken from 6 patients were prepared for further comparative analyses of biomarker expression levels.

The fluorescence levels of the studied biomarkers were assessed by protein microarray technology using the Quantibody Human Angiogenesis Array 1000 kit (RayBiotech, Norcross, GA, USA). Tissue homogenisation was performed in T-PER Tissue Protein Extraction Reagent with EDTA, protease inhibitor cocktail (Heat^{™} Protease Inhibitor Coctail × 100) and 0.5 mM PMSF. Total protein levels were determined using bicinchoninic acid (Pierce BCA Protein Assay Kit) and NanoDrop1000 (Thermo Scientific). All protein samples were diluted in PBS to contain 0.5 mg/ml of total protein and 100 pl samples were added to each well of the slides. All steps were performed at room temperature according to the manufacturer's instructions and original reagents (RayBiotech). The procedure for reading the arrays and concentrations of the respective proteins was performed according to the manufacturer's instructions. Statistical analysis of the results was performed using GraphPad Prism software version 8.1.2 (GraphPad Software Inc., San Diego, CA, USA). Before selecting a statistical test for each dataset, an independent normality test of the distribution was performed. Based on the results obtained with the Kolmogorov-Smirnov test, the optimal test was decided upon. The coefficient of statistical significance was set at α < 0.05 (two-sided test).

### Example 2 - Experimental results

Depending on whether the normality criterion for the data distribution between the study groups was met, parametric (paired t test) or non-parametric (Wilcoxon test for paired observations) tests were used to compare the protein expression levels. The EV isolates showed significantly higher concentrations for Ang-1 protein in normal control tissue than in cancer (P=0.0290; Wilcoxon test). Ang-1 levels estimated in the prepared tissue lysates were not significantly different between the study groups (P=0.8826; paired t test). Concentrations of other biomarkers showed significantly higher expression levels in the CRC-derived extracellular vesicles than those obtained from surgical margins representing normal colon wall and mucosa: for TIMP-1 (P<0.0001; Wilcoxon test), TIMP-2 (P=0.0003; Wilcoxon test), IL-8 (P=0.0002; Wilcoxon test), GRO-α(P=0.0001; Wilcoxon test) and HGF (P=0.0003; paired t test). Again, a clear disparity was observed between the biomarker concentrations assessed in the EVs and the biomarker levels assessed in the prepared tissue lysates, showing that there were more pronounced differences in the expression of the proteins tested in the vesicles than in the tissue homogenates. The expression level of TIMP-1 in the tissue lysates was, as in the EVs, significantly higher in the tumour tissue (P=0.0010; paired t test). Significant differences were also shown for interleukin 8 (IL-8) with higher expression of this cytokine in cancer (P=0.0313; Wilcoxon test). In contrast, the concentration levels of the other biomarkers in the tissue lysates did not show significant statistical differences between the margin-cancer groups tested: TIMP-2 (P=0.2570; paired t test), GRO-α(P=0.0625; Wilcoxon test) and HGF (P=0.3125; Wilcoxon test). Comparative analyses for the biomarkers tested in the EVs and in the tissue fragment lysates are presented graphically in the drawing using bar charts.

### Conclusions:

The study confirms the existence of statistically significant differences in the isolated extracellular vesicles/exosomes for the expression of Ang-1, TIMP-1, TIMP-2, IL8, GRO-α and HGF between colorectal cancer and normal controls. Evaluation of the concentrations of the biomarkers tested in extracellular vesicles/exosomes is more accurate than their evaluation in the respective tissue homogenates. CRC tumour cells release extracellular vesicles that are detectable in physiological fluids such as blood and in serum and plasma isolates. Biomarker assessment performed only in the extracellular vesicles yields more pronounced differences in the levels of the aforementioned biomarkers than their analysis in the lysates of complete tissue fragments containing extracellular vesicles, cells and other tissue components.

### Literature:

[1] Yáñez-Mó M et al. Biological properties of extracellular vesicles and their physiological functions. J Extracell Vesicles. 2015 May 14;4:27066. doi: 10.3402/jev.v4.27066. eCollection 2015.
[2] Zadka L, Grybowski DJ, Dzi 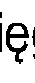 giel P. Modeling of the immune response in the pathogenesis of solid tumors and its prognostic significance. Cell Oncol (Dordr). 2020 Aug;43(4):539-575. doi: 10.1 007/s13402-020-00519-3.
[3] Soekmadji C, Li B, Huang Y, Wang H, An T, Liu C, Pan W, Chen J, Cheung L, Falcon-Perez JM, Gho YS, Holthofer HB, Le MTN, Marcilla A, O'Driscoll L, Shekari F, Shen TL, Torrecilhas AC, Yan X, Yang F, Yin H, Xiao Y, Zhao Z, Zou X, Wang Q, Zheng L. The future of Extracellular Vesicles as Theranostics - an ISEV meeting report. J Extracell Vesicles. 2020 Sep 4;9(1):1809766. doi:10.1080/20013078.2020.1809766.
[4] Choi D, Spinelli C, Montermini L, Rak J. Oncogenic Regulation of Extracellular Vesicle Proteome and Heterogeneity. Proteomics. 2019 Jan;19(1-2):e1800169. doi: 10.1002/pmic.201800169.
[5] Lafitte M, Lecointre C, Roche S. Roles of exosomes in metastatic colorectal cancer. Am J Physiol Cell Physiol. 2019; 317: 869-880.
[6] Rak J. Cancer: organ-seeking vesicles. Nature. 2015; 527: 312-314.
[7] Soborczyk A, Deptala A. Markery nowotworowe w praktyce klinicznej. Choroby Serca i Naczy 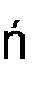 2007;4(4):184-189.
[8] Zheng X, Xu K, Zhou B, Chen T, Huang Y, Li Q, Wen F, Ge W, Wang J, Yu S, Sun L, Zhu L, Liu W, Gao H, Yue L, Cai X, Zhang Q, Ruan G, Zhu T, Wu Z, Zhu Y, Shao Y, Guo T, Zheng S. A circulating extracellular vesicles-based novel screening tool for colorectal cancer revealed by shotgun and data-independent acquisition mass spectrometry. J Extracell Vesicles. 2020 Apr 14;9(1):1750202. doi: 10.1080/20013078.2020.1750202. eCollection 2020.
[9] Zadka L, Piotrowska A, Opali 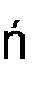 ska A, Haczkiewicz-Le 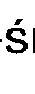 niak K, Grybowski D, Ceremuga I, Chabowski M, Dziegiel P. Comparative analysis of exosome markers and extracellular vesicles between colorectal cancer and cancer-associated normal colonic mucosa. Pol Arch Intern Med. 2020 Aug 27;130(7-8):640-648. doi: 10.20452/pamw.15462. Epub 2020 Jun 26.
[10] Zadka L, Buzalewicz I, Ulatowska-Jarza A, Rusak A, Kochel M, Ceremuga I, Dziegiel P. Label-Free Quantitative Phase Imaging Reveals Spatial Heterogeneity of Extracellular Vesicles in Selected Colon Disorders. Am J Pathol. 2021 Aug 21:S0002-9440(21)00353-9. doi: 10.1016/j.ajpath.2021.08.005. Online ahead of print.
[11] Chang LC, Hsu YC, Chiu HM, Ueda K, Wu MS, Kao CH, Shen TL. Exploration of the Proteomic Landscape of Small Extracellular Vesicles in Serum as Biomarkers for Early Detection of Colorectal Neoplasia. Front Oncol. 2021 Sep 13;11:732743. doi: 10.3389/fonc.2021.732743. eCollection 2021.
[12] Freire MO, Van Dyke TE (2013) Natural resolution of inflammation. Periodontol 2000 Oct;63(1):149-64. doi: 10.1111/prd.12034.
[13] Kurzyk A (2015) [Angiogenesis - possibilities, problems and perspectives]. Postepy Biochem 61(1):25-34.
[14] Kazerounian S, Lawler J (2017) Integration of pro- and anti-angiogenic signals by endothelial cells. J Cell Commun Signal Dec 20. doi: 10.1007/s12079-017-0433-3.
[15] Cohen MM Jr (2009) Judah Folkman, MD, 1933-2008: father of angiogenesis. J Craniofac Surg Mar;20 Suppl 1:590-1. doi: 10.1097/SCS.0b013e3181927e8a.
[16] Folkman J (1971) Tumor angiogenesis: therapeutic implications. N Engl J Med Nov 18;285(21):1182-6.
[17] Cheong, Y. C. and Ledger, W. L. (2003), Cytokines in health and disease. The Obstetrician & Gynaecologist, 5: 155-159. doi:10.1576/toag.5.3.155
[18] Jandl C, Loetsch C, King C (2017) Cytokine Expression by T Follicular Helper Cells. Methods Mol Biol 1623: 95-103. doi: 10.1007/978-1-4939-7095-7_8.
[19] Shirota H, Klinman DM, Ito SE, Ito H, Kubo M, Ishioka C (2017) IL4 from T Follicular Helper Cells Downregulates Antitumor Immunity. Cancer Immunol Res Jan;5(1):61-71. doi: 10.1158/2326-6066.CIR-16-0113. Epub 2016 Dec 5.
[20] Wang H, Yang X (2017) Association between serum cytokines and progression of breast cancer in Chinese population. Medicine (Baltimore) Dec; 96 (49): e8840. doi: 10.1097/MD.0000000000008840.
[21] Grivennikov SI, Karin M. (2011) Inflammatory cytokines in cancer: tumour necrosis factor and interleukin 6 take the stage. Ann Rheum Dis Mar;70 Suppl 1: i104-8. doi: 10.1136/ard.2010.140145.
[22] Wang G, Huang J, Zhu H, Ju S, Wang H, Wang X. Overexpression of GRO-β is associated with an unfavorable outcome in colorectal cancer. Oncol Lett. 2016 Apr;11(4):2391-2397. doi: 10.3892/ol.2016.4222. Epub 2016 Feb 10.
[23] Baier PK, Eggstein S, Wolff-Vorbeck G, Baumgartner U, Hopt UT. Chemokines in human colorectal carcinoma. Anticancer Res. 2005 Sep-Oct;25(5):3581-4.
[24] Altadill A, Eiro N, González LO, Andicoechea A, Fernández-Francos S, Rodrigo L, Garcia-Muñiz JL, Vizoso FJ. Relationship between Metalloprotease-7 and -14 and Tissue Inhibitor of Metalloprotease 1 Expression by Mucosal Stromal Cells and Colorectal Cancer Development in Inflammatory Bowel Disease. Biomedicines. 2021 Apr 30;9(5):495. doi: 10.3390/biomedicines9050495.
[25] Stidham RW, Higgins PDR. Colorectal Cancer in Inflammatory Bowel Disease. Clin Colon Rectal Surg. 2018 May;31(3):168-178. doi: 10.1055/s-0037-1602237. Epub 2018 Apr 1.
[26] Huang X, Lan Y, Li E, Li J, Deng Q, Deng X. Diagnostic values of MMP-7, MMP-9, MMP-11, TIMP-1, TIMP-2, CEA, and CA19-9 in patients with colorectal cancer. J Int Med Res. 2021
[27] Huang CY, Zhou QY, Hu Y, Wen Y, Qiu ZW, Liang MG, Mo JL, Xu JH, Sun C, Liu FB, Chen XL. Hepatocyte growth factor is a prognostic marker in patients with colorectal cancer: a meta-analysis. Oncotarget. 2017 Apr4;8(14):23459-23469. doi: 10.18632/oncotarget.15589.
[28] Davis S, Aldrich TH, Jones PF, Acheson A, Compton DL, Jain V, Ryan TE, Bruno J, Radziejewski C, Maisonpierre PC, Yancopoulos GD. Isolation of angiopoietin-1, a ligand for the TIE2 receptor, by secretion-trap expression cloning. Cell. 1996 Dec 27;87(7):1161-9. doi: 10.1016/s0092-8674(00)81812-7.
[29] Hong S, Jung HI, Ahn TS, Kim HJ, Lee KT, Baek MJ, Bae SB. Expressions and Clinical Significances of Angiopoietin-1, Angiopoietin-2, and Tie-2 Receptor in Patients With Colorectal Cancer. Ann Coloproctol. 2017 Feb;33(1):9-15. doi: 10.3393/ac.2017.33.1.9. Epub 2017 Feb 28.
[30] Engin H, Üstünda 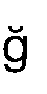 Y, Tekin iÖ, Gökmen A, Ertop 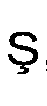 , ilikhan SU. Plasma concentrations of angiopoietin-1, angiopoietin-2 and Tie-2 in colon cancer. Eur Cytokine Netw. 2012 Jun;23(2):68-71. doi: 10.1684/ecn.2012.0308.
[31] Abad MC, Arni RK, Grella DK, Castellino FJ, Tulinsky A, Geiger JH. The X-ray crystallographic structure of the angiogenesis inhibitor angiostatin. J Mol Biol. 2002 May 10;318(4):1009-17. doi: 10.1016/S0022-2836(02)00211-5.
[32] Shantha Kumara HM, Tohme ST, Yan X, Nasar A, Senagore AJ, Kalady MF, Hyman N, Kim IY, Whelan RL. Plasma levels of angiostatin and endostatin remain unchanged for the first 3 weeks after colorectal cancer surgery. Surg Endosc. 2011 Jun;25(6):1939-44. doi: 10.1007/s00464-010-1491-2. Epub 2010 Dec 22.
[33] Ertekin T, Ekinci N, Karaca O, Nisari M, Canoz O, Ulger H. Effect of angiostatin on 1,2-dimethylhydrazine-induced colon cancer in mice. Toxicol Ind Health. 2013 Jul;29(6):490-7. doi: 10.1177/0748233712440137. Epub 2012 Mar 5.

## Claims

1. A set of protein biomarkers for *in vitro* diagnosis of early stage colorectal cancer or colorectal cancer, **characterised in that** it comprises TIMP-1, TIMP-2, IL8, GRO-α, HGF and Ang-1.

2. The set of biomarkers of claim 1, **characterised in that** the expression value of any biomarker from the group comprising TIMP-1, TIMP-2, IL8, GRO-α, HGF, Ang-1 is determined in extracellular vesicles.

3. A method for *in vitro* diagnosis of early stage colorectal cancer or colorectal cancer, **characterised in that** it comprises the following steps:
a) a biological sample in the form of tissue material is obtained from the patient,
b) extracellular vesicles are isolated from the biological sample obtained from the patient,
c) in the isolated extracellular vesicles, the expression levels of at least two protein biomarkers selected from a set including TIMP-1, TIMP-2, IL8, GRO-α,

4. The method of claim 3, **characterised in that** the tissue material is solid tissue or body fluid.

5. The method of claim 4, **characterised in that** the body fluid may be blood, plasma, serum, saliva or urine.

6. The method of claim 3, **characterised in that** the extracellular vesicles are exomers, exosomes, ectosomes, microvesicles, apoptotic bodies or oncosomes.

7. The method of claim 3, **characterised in that** the isolation of extracellular vesicles is carried out by centrifugation, ultracentrifugation, chromatography or by using kits for precipitation of extracellular vesicles.

8. The method of claim 3, **characterised in that** ELISA immunoassay method, immunofluorescence assay method, Western blot (immunoblot) method, flow cytometry method, flow nanocytometry method, protein microarray method or detection using biosensors is used to evaluate the biomarker expression level.

9. The method of claim 3, **characterised in that** the expression levels of the TIMP-1, TIMP-2, IL8, GRO-α and HGF biomarkers correspond to their elevated concentrations in a patient with early stage colorectal cancer or colorectal cancer than in a healthy person.

10. The method of claim 3, **characterised in that** the expression level of the Ang-1 biomarker corresponds to its reduced concentration in a patient with early stage colorectal cancer or colorectal cancer than in a healthy person.
